(19) **Europäisches Patentamt / European Patent Office / Office européen des brevets**

(11) **EP 3 038 261 B1**

(12) **FASCICULE DE BREVET EUROPEEN**

(45) Date de publication et mention de la délivrance du brevet:
**15.07.2020 Bulletin 2020/29**

(51) Int Cl.:
*H03M 7/30* (2006.01)    *A61B 5/04* (2006.01)

(21) Numéro de dépôt: **15200245.7**

(22) Date de dépôt: **15.12.2015**

(54) **PROCÉDÉ DE PRÉTRAITEMENT DE COMPRESSION DE DONNÉES ADAPTÉ À DES DONNÉES DE MESURES DE SIGNAUX ÉLECTRO-CORTICOGRAPHIQUES (ECOG) ET SYSTÈME D'ACQUISITION ET DE TRANSMISSION DE DONNÉES ECOG**

VERFAHREN ZUR KOMPRESSIONSAUFBEREITUNG VON DATEN, DAS ANGEPASST IST AN ELEKTROKORTIKOGRAFISCHE SIGNALMESSDATEN (ECOG), UND SYSTEM ZUR ERFASSUNG UND ÜBERTRAGUNG VON ECOG-DATEN

METHOD OF DATA COMPRESSION PREPROCESSING TAILORED TO DATA OF MEASUREMENTS OF ELECTRO-CORTICOGRAPHIC SIGNALS (ECOG) AND SYSTEM FOR ACQUIRING AND TRANSMITTING ECOG DATA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2014 FR 1463136**

(43) Date de publication de la demande:
**29.06.2016 Bulletin 2016/26**

(73) Titulaire: **COMMISSARIAT À L'ÉNERGIE ATOMIQUE ET AUX ÉNERGIES ALTERNATIVES 75015 Paris (FR)**

(72) Inventeurs:
- **FOERSTER, Michael 33170 Gradignan (FR)**
- **DEHAENE, David 91120 Palaiseau (FR)**

(74) Mandataire: **Tanguy, Yannick Marks & Clerk France Conseils en Propriete Industrielle Immeuble Visium 22, avenue Aristide Briand 94117 Arcueil Cedex (FR)**

(56) Documents cités:
**WO-A2-2008/015449**

- **SCHMALE S ET AL: "Exploiting correlation in neural signals for data compression", 2014 22ND EUROPEAN SIGNAL PROCESSING CONFERENCE (EUSIPCO), EURASIP, 1 septembre 2014 (2014-09-01), pages 2080-2084, XP032682013,**
- **TAEHOON KIM ET AL: "Spatiotemporal compression for efficient storage and transmission of high-resolution electrocorticography data", THE EFFECT OF APPLIED COMPRESSIVE LOADING ON TISSUE-ENGINEERED CARTILAGE CONSTRUCTS CULTURED WITH TGF-BETA3, IEEE, 28 août 2012 (2012-08-28), pages 1012-1015, XP032463090, ISSN: 1557-170X, DOI: 10.1109/EMBC.2012.6346105**
- **FAN ZHANG ET AL: "A Low-Power ECoG/EEG Processing IC With Integrated Multiband Energy Extractor", IEEE TRANSACTIONS ON CIRCUITS AND SYSTEMS I: REGULAR PAPERS, vol. 58, no. 9, 1 septembre 2011 (2011-09-01), pages 2069-2082, XP055207206, ISSN: 1549-8328, DOI: 10.1109/TCSI.2011.2163972**
- **CAPURRO IGNACIO ET AL: "Low-complexity, multi-channel, lossless and near-lossless EEG compression", 2014 22ND EUROPEAN SIGNAL PROCESSING CONFERENCE (EUSIPCO), EURASIP, 1 September 2014 (2014-09-01), pages 2040-2044, XP032682074, [retrieved on 2014-11-11]**

• TAEHOON KIM ET AL.: "Spatiotemporal compression for efficient storage and transmission of high-resolution electrocorticography data", PROCEEDINGS OF ENGINEERING IN MEDICINE AND BIOLOGY SOCIETY (EMBC), 2012 ANNUAL INTERNATIONAL CONFERENCE OF THE IEEE, 28 August 2012 (2012-08-28), pages 1012-1015,

**Description**

La présente invention concerne un procédé de prétraitement de compression de données adapté à des données brutes $u_i(t_N)$ de mesures de signaux électrocorticographiques évoluant dans le temps, et un système de mise en œuvre d'un tel procédé.

L'invention concerne également un programme d'ordinateur mettant en œuvre le procédé et mis en œuvre par le système selon l'invention.

Dans le domaine de l'acquisition de signaux cérébraux à la surface du cortex d'un animal ou d'un être humain, dénommé « patient », il est connu et largement répandu d'acquérir les signaux au moyen d'un système de mesure d'ElectroCorticoGramme (ECoG).

Les systèmes d'acquisition de signaux biologiques cérébraux existent depuis longtemps, mais ce n'est que depuis peu que la miniaturisation des électrodes et de l'électronique a permis de développer des systèmes d'acquisition sans fil. Les systèmes filaires classiques, comme les bonnets de mesure électroencéphalographique EEG par exemple, lient physiquement le ou les capteurs à proximité ou en contact direct avec le cortex d'un patient à une électronique de mesure, qui est à son tour reliée à une unité de traitement des données, par exemple un ordinateur.

Les systèmes sans fil, par contre qui conviennent particulièrement à l'utilisation d'électrodes en contact direct avec la surface d'un cortex, intègrent l'électronique sur le lieu de la mesure et transmettent les données à l'unité de traitement des données par un moyen de communication sans fil.

C'est pourquoi, pour de tels systèmes sans fil, la diminution du volume des données brutes acquises, c'est-à-dire leur compression, revêt un intérêt encore plus important car elle permet de réduire les contraintes pesant sur les modules de transmission de données sans fil ou sur les supports de stockage des données. Par extension, elle réduit également les besoins en énergie des systèmes d'acquisition de signaux cérébraux et permet donc de développer des systèmes dont les performances sont plus proches des systèmes filaires car moins de compromis de conception sont à faire.

De manière générale, la compression de données est un domaine technique dans lequel pour de multiples applications, comme la compression de texte, d'image, de son ou de vidéos, de nombreuses recherches ont été effectuées ou sont en cours.

Les approches utilisées pour réduire la quantité de données dépendent fortement de l'application. Ainsi, pour de la compression de texte, sont fréquemment utilisées des approches fondées sur un dictionnaire, en anglais « dictionary-based », où des mots sont substitués par leur emplacement dans un dictionnaire, pour la compression d'image ou de son sera réalisée une compression avec pertes en supprimant les détails inaudibles, et pour la compression vidéo, en plus de supprimer les détails invisibles à l'œil sera tiré profit de la similitude entre deux images successives.

Il existe cependant des méthodes communes, largement utilisées, qui permettent de réaliser la compression de données en tirant profit des propriétés statistiques des données à compresser. Le codage de Huffman et le codage arithmétique sont les plus utilisées de ces méthodes. Ces techniques interviennent souvent en complément d'une technique de compression adaptée à l'application.

En somme, un algorithme de compression appliquera souvent un prétraitement adapté au type de données à traiter, avant de les coder en utilisant des techniques génériques.

Dans le cas d'un algorithme de compression appliqué à des données de mesures ECoG, le procédé de compression comporte un prétraitement de compression adapté à ce type de données, dénommée par la suite prétraitement de compression ou prétraitement, suivi d'une compression entropique générique classique indépendante de l'application, dénommée par la suite encodage entropique.

Dans le domaine de la compression des données ECoG, l'article de Taehoon Kim et al., intitulé « Spatiotemporal compression for efficient storage and transmission of high-resolution electrocorticography data", et publié dans Proceedings of Engineering in Medicine and Biology Society (EMBC), 2012 Annual International Conférence of the IEEE, pp. 1012-1015, Aug. 28, 2012- Sept. 1, 2012, décrit l'application de procédés de prétraitement issus de la compression d'images vidéo à la compression de données d'ElectroCorticoGramme ECoG. L'article de Schmale S. et al., intitulé « Exploiting correlation in neural signals for data compression », et publié dans 2014 22nd European Signal Processing Conférence, EURASIP, 1 septembre 2014, pages 2080-2084, la demande de brevet WO 2008/015449, et l'article de Capurro Ignacio et al., intitulé « Low-complexity, multichannel, lossless and near-lossless EEG compression, et publié dans 2014 22nd European Signal Processing Conférence, EURASIP, 1 septembre 2014, pages 2040-2044, décrivent chacun un procédé de compression de données de mesures spatiotemporelles de signaux électrocorticographiques qui exploite la corrélation spatiale et temporelle des signaux neuronaux. Les traitements choisis exigent beaucoup de ressources de calcul et ne sont pas sans présenter des pertes d'information.

Le problème technique est de fournir un procédé de prétraitement de compression de données de mesures d'ElectroCorticoGramme (ECoG) évoluant dans le temps qui est sans pertes d'information de l'ElectroCorticoGramme, qui suit fidèlement avec un faible retard l'évolution temporelle de l'ElectroCorticoGramme (exigence de temps réel), et qui utilise un minimum de ressources de calcul.

De manière correspondante, le problème technique est de fournir une unité de prétraitement de compression de données de mesures d'ElectroCorticoGramme (ECoG) évoluant dans le temps qui est sans pertes d'information de l'ElectroCorticoGramme, qui suit fidèlement avec un faible retard l'évolution temporelle de l'ElectroCorticoGramme, et qui utilise un minimum de ressources de calcul, et un système d'acquisition et de transmission comportant un tel compresseur.

L'invention est définie par le jeu des revendications.

L'invention sera mieux comprise à la lecture de la description de plusieurs formes de réalisation qui va suivre, donnée uniquement à titre d'exemple et faite en se référant aux dessins sur lesquels :

- la Figure 1 est une vue d'un exemple d'architecture d'un système d'acquisition de mesures ECoG et de transmission de données de mesures ECoG compressées selon l'invention ;
- les Figures 2A, 2B, 2C, 2D sont des illustrations d'exemples de fonctions de voisinage $\sigma_i$, pouvant être utilisées pour la mise en œuvre d'un procédé de prétraitement de compression de données brutes de mesures ECoG selon l'invention ;
- la Figure 3 est un ordinogramme d'un procédé de prétraitement de compression de données brutes de mesures ECoG selon l'invention ;
- La Figure 4 est une vue des performances entropiques d'un procédé de compression utilisant seulement un encodage entropique classique et n'utilisant pas de prétraitement de compression de données brutes de mesures ECoG ;
- La Figure 5 est une vue des performances entropiques d'un procédé de compression utilisant un prétraitement de compression selon l'invention, appliqué à des données brutes de mesures ECoG, suivie d'un encodage entropique générique ;
- La Figure 6 est un ordinogramme d'un procédé de décompression mis en œuvre par le décompresseur du système d'acquisition et de transmission de la Figure 1 et correspondant à un procédé de prétraitement de compression selon l'invention.

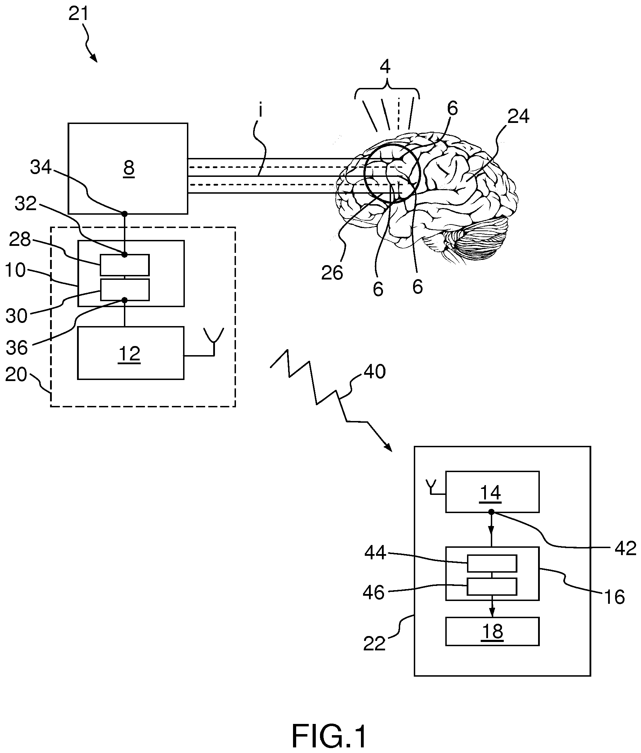

Suivant la Figure 1, un système 2 d'acquisition et de transmission de données de mesures de signaux électrocorticographiques évoluant dans le temps comprend un ensemble ou une matrice 4 d'électrodes corticales 6 d'acquisition de signaux bruts électrocorticographiques, une unité d'échantillonnage 8 des signaux bruts en des données brutes, un compresseur 10 des données brutes en des données compressées à transmettre, un émetteur 12, un récepteur 14 et un décompresseur de données transmises 16, et une unité de traitement 18 des données de mesures brutes restituées.

Le compresseur 10 et l'émetteur 12 sont intégrés dans une unité d'émission tandis que le récepteur 14, le décompresseur 16 et l'unité de traitement 18 sont intégrés dans une unité de réception 22.

Les électrodes corticales 6 de l'ensemble ou de la matrice 4 sont disposées ici en contact direct du cortex 24 d'un patient dans une zone d'observation 26.

En variante, les électrodes corticales sont disposées à proximité de la surface du cortex.

Suivant la Figure 1, les électrodes corticales 6 sont caractérisées chacune par un indice entier i différent d'identification et une position r(i) à la surface du cortex, l'indice i variant de 1 à L, et L désignant le nombre total d'électrodes 6.

Les électrodes corticales 6 sont configurées chacune pour acquérir un signal temporel électrocorticographique brut désigné par $u_i(t)$ en fonction de l'indice i de l'électrode observée.

L'unité d'échantillonnage 8 est configurée pour échantillonner à des instants successifs $t_N$, N étant un entier désignant le rang de succession d'un échantillon d'un signal dans le temps, les signaux brutes $u_i(t)$ et coder ici en binaire leur amplitude en des données brutes associées.

De manière préférée l'échantillonnage est effectué périodiquement, et de manière générale le codage de l'amplitude est réalisé dans une base quelconque.

Le compresseur de données 10 comporte une unité de prétraitement de compression 28 suivi d'un encodeur entropique 30.

L'unité de prétraitement de compression 28 possède un port d'entrée 32 connecté à un port de sortie 34 de l'unité d'échantillonnage 8, et il est configuré pour convertir les données brutes $u_i(t)$ en des données prétraitées $\varepsilon_i(t_N)$ à entropie moins élevée suivant un procédé de prétraitement de compression de l'invention.

L'émetteur de transmission 12 est connecté à un port de sortie 36 du compresseur 10 des données brutes $u_i(t)$, et est configuré pour transmettre les données prétraitées $\varepsilon_i(t_N)$ et encodées successivement par l'encodeur entropique 30, au travers d'une liaison 22 ici sans fil, c'est-à-dire radioélectrique.

Le récepteur de transmission 14, distant de l'émetteur de transmission 12, est configuré pour recevoir les données prétraitées $\varepsilon_i(t_N)$ et encodées successivement par l'encodeur entropique 30, et les restituer sans erreur au décompresseur 16, connecté à un port de sortie 42 du récepteur de transmission 14.

Le décompresseur 16 comporte un décodeur entropique 44 suivi d'un décompresseur de prétraitement 46.

Le décodeur entropique 44, connecté au port de sortie 42 du récepteur de transmission 14 et dont l'algorithme de décompression générique correspond à l'algorithme de compression générique de l'encodeur entropique 30, est configuré pour restituer en entrée du décompresseur de prétraitement 46 les données prétraitées $\varepsilon_i(t_N)$ ou signaux d'erreur.

Le décompresseur de prétraitement 46 est configuré pour décompresser les données prétraitées $\varepsilon_i(t_N)$ en les données brutes $u_i(t)$ suivant un algorithme de décompression de prétraitement correspondant à l'algorithme de prétraitement de compression.

L'unité de prétraitement de compression 28 est configurée de manière générale pour mettre en œuvre un procédé de prétraitement de compression qui comporte une étape de prétraitement de compression effective des données brutes.

Dans cette étape de prétraitement de compression effective des données brutes $u_i(t)$, pour i variant de 1 à L et à chaque instant d'échantillonnage successifs $t_N$ à partir d'un rang $N_0$, le signal brut $u_i(t)$ acquis par l'électrode i est transformé en un signal prétraité ou signal d'erreur $\varepsilon_i(t_N)$, égal à la différence entre un premier terme et un deuxième terme.

Le premier terme est égal au signal brut acquis en l'électrode i à l'instant courant $t_N$, ou de rang N.

Le deuxième terme est une fonction de prédiction fi inversible qui dépend de signaux bruts observés dans un passé proche jusqu'à l'instant $t_{N-p}$, p désignant un rang de passé le plus lointain par rapport au rang N de l'instant courant $t_N$, au moins en un nombre entier m d'électrodes voisines $\sigma_i(j)$ de l'électrode observée i et au plus en le nombre entier m d'électrodes voisines $\sigma_i(j)$ de l'électrode observée et en l'électrode observée i, $\sigma_i$ étant une fonction de voisinage de l'électrode i observée qui pour j variant de 1 à m associe l'indice $\sigma_i(j)$ d'une électrode voisine de l'électrode i observée et dont le signal est corrélé à celui de l'électrode observé i.

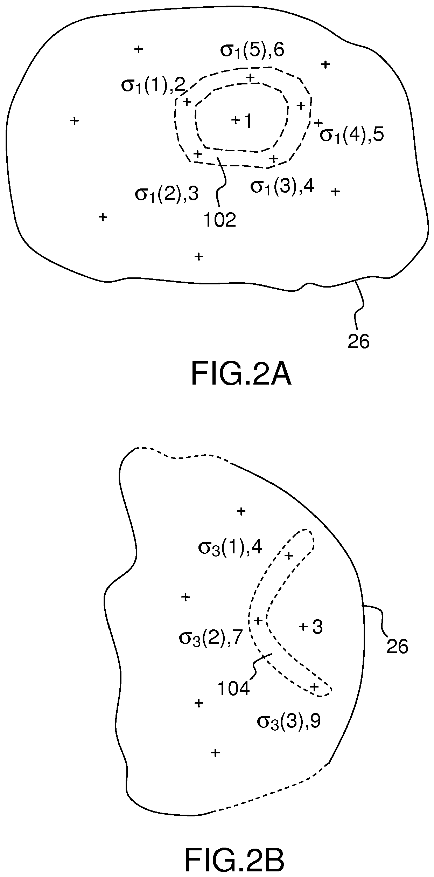

Suivant la Figure 2A et un premier exemple d'illustration d'une fonction de voisinage $\sigma_i$, une électrode i observée est entourée ici de 5 électrodes immédiatement voisines suivant une unique couronne 102 illustrée en traits pointillés.

Ici, l'électrode i, observée et représentée à titre d'exemple sur la Figure 2A, est l'électrode 1 pour laquelle i est égale à 1. Les électrodes immédiatement voisines sont au nombre de m(1) électrodes, m(1) étant ici égale à 5, les indices d'identification de ces électrodes $\sigma_1(1)$, $\sigma_1(2)$, $\sigma_1(3)$, $\sigma_1(4)$, $\sigma_1(5)$ étant pour cet exemple respectivement égaux à 2, 3, 4, 5, 6, cette correspondance étant définie au travers de la fonction de voisinage $\sigma_1$.

Suivant la Figure 2B et un deuxième exemple d'illustration d'une fonction de voisinage $\sigma_i$, une électrode i observée, située en bordure de la zone 26 de surface corticale couverte par l'ensemble des électrodes, est entourée partiellement par des électrodes immédiatement voisines formant un arc 104 de couronne.

Ici, l'électrode i observée et représentée à titre d'exemple sur la Figure 2B est l'électrode 3 pour laquelle i est égale à 3. Les électrodes immédiatement voisines sont au nombre de m(3) électrodes, m(3) étant ici égale à 3, les indices d'identification de ces électrodes, $\sigma_3(1)$, $\sigma_3(2)$, $\sigma_3(3)$, étant respectivement égaux à 4, 7 et 9, cette correspondance étant définie au travers de la fonction de voisinage $\sigma_3$.

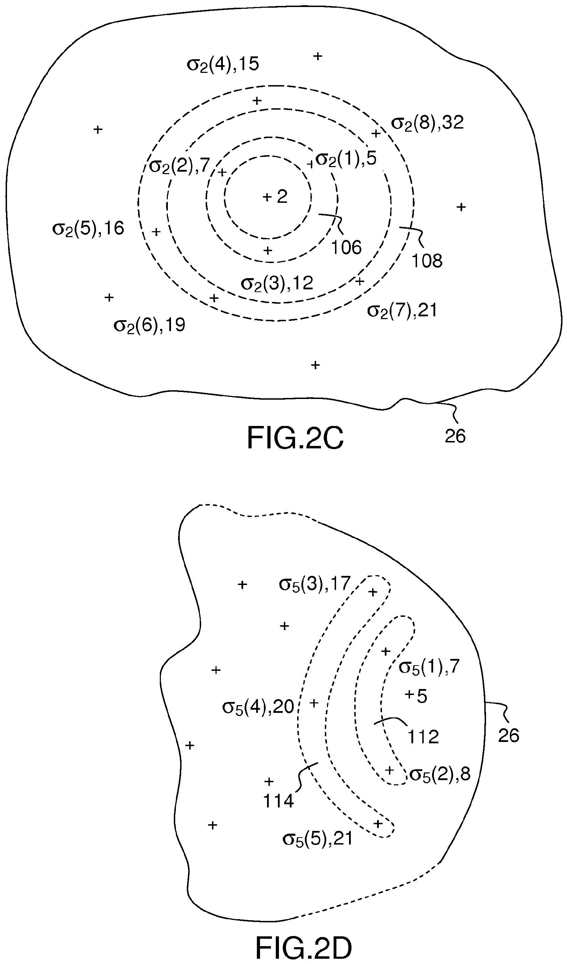

Suivant la Figure 2C et un troisième exemple d'illustration d'une fonction de voisinage $\sigma_i$, une électrode i observée est entourée d'électrodes voisines formant deux couronnes 106 et 108.

Ici, l'électrode i, observée et représentée à titre d'exemple sur la Figure 2C, est l'électrode 2 pour laquelle i est égale à 2. Les électrodes voisines sont au nombre de m(2) électrodes, m(2) étant ici égal à 8, les indices d'identification de ces électrodes $\sigma_2(1)$, $\sigma_2(2)$, $\sigma_2(3)$, $\sigma_2(4)$, $\sigma_2(5)$, $\sigma_2(6)$, $\sigma_2(7)$, $\sigma_2(8)$, étant respectivement égaux à 5, 7, 12, 15, 16, 19, 21, 32, cette correspondance étant définie au travers de la fonction de voisinage $\sigma_2$, les électrodes 5, 7, 12 formant une première couronne 106, illustré en traits pointillés, d'électrodes immédiatement voisines et entourant l'électrode observée 2, et les électrodes 15, 16, 19, 21, 32, formant une deuxième couronne 108, illustrée en traits pointillés, d'électrodes immédiatement voisines et entourant la première couronne d'électrodes 102.

Suivant la Figure 2D et un quatrième exemple d'illustration d'une fonction de voisinage $\sigma_i$, une électrode i observée, située en bordure de la zone 24 de surface corticale couverte par l'ensemble des L électrodes, est entourée partiellement par des électrodes formant deux arcs de couronne.

Ici, l'électrode i observée et représentée à titre d'exemple sur la Figure 2D est l'électrode 5 pour laquelle i est égale à 5. Les électrodes voisines sont au nombre de m(5) électrodes, m(5) étant ici égal à 5, les indices d'identification de ces électrodes $\sigma_5(1)$, $\sigma_5(2)$, $\sigma_5(3)$, $\sigma_5(4)$, $\sigma_5(5)$, étant respectivement égaux à 7, 8, 17, 20 et 21, cette correspondance étant définie au travers de la fonction de voisinage $\sigma_5$, les électrodes 7, 8 formant un premier arc 112 de couronne, illustré en traits pointillés, d'électrodes immédiatement voisines et entourant partiellement l'électrode observée 5, et les électrodes 17, 20, 21, formant un deuxième arc 114 de couronne, illustré en traits pointillés, d'électrodes immédiatement voisines de et entourant le premier arc 112 de couronne d'électrodes.

Suivant un premier mode de réalisation du procédé de prétraitement de compression, pour chaque électrode i observée, la fonction de prédiction fi du deuxième terme du signal prétraité est une première fonction $f1_i$ de prédiction inversible qui dépend exclusivement des signaux bruts observés dans le passé proche jusqu'à l'instant $t_{N-p(i)}$, en le nombre entier m(i) d'électrodes voisines $\sigma_i(j)$ de l'électrode observée i.

Dans ce cas, l'expression du signal prétraité s'écrit :

$$\varepsilon_i(t_N) = u_i(t_N) - f1_i \left( \left( u_{\sigma_i(j)}(t_{N-k}) \right)_{\substack{j \in [1,m(i)] \\ k \in [1,p(i)]}} \right)$$

Equation 1,

dans laquelle :

.- $t_N$ désigne un instant d'échantillonnage courant et N désigne le rang courant d'échantillonnage associé ;

.- i est l'indice de l'électrode observée ;

.- $u_i(t_N)$ représente le signal brut observé en l'électrode observée i à l'instant d'échantillonnage courant $t_N$ ;

.- $\varepsilon_i(t_N)$ représente le signal prétraité ou signal d'erreur en l'électrode observée i à l'instant d'échantillonnage courant $t_N$ ;

.- m(i) est le nombre total d'électrodes d'un voisinage pertinent d'influence sur l'électrode observée i, c'est-à-dire des électrodes voisines dont les signaux bruts $u_{\sigma_i(j)}(t_{N-k})$ influencent et contribuent au signal brut de l'électrode observée i $u_i(t_N)$ à l'instant $t_N$ ;

.- $\sigma_i$ est une fonction de voisinage définissant une correspondance biunivoque entre un indice j de parcours des électrodes du voisinage pertinent et les indices d'identification de ces électrodes au sein de l'ensemble total des L électrodes d'acquisition ;

.- j est l'indice de parcours de la fonction de voisinage $\sigma_i$ pertinente de l'électrode observée i, j variant de 1 à m(i) ;

.- p(i) désigne une profondeur de mémoire ou du rang de passé le plus lointain des échantillons passés $u_{\sigma_i(j)}(t_{N-k})$ des signaux bruts du voisinage pertinent de l'électrode observée i ayant une influence sur le signal brut $u_i(t_N)$ ayant le rang courant N d'échantillonnage de l'électrode observée i, p(i) dépendant de l'indice i le cas échéant.

.- k désigne un indice relatif de parcours et de remontée vers le passé des échantillons passés ayant une influence sur ou une contribution à l'échantillon courant $u_i(t_N)$ du signal brut observé de rang courant N.

Suivant un deuxième mode de réalisation du procédé de prétraitement de compression, pour chaque électrode i observée, la fonction de prédiction fi du deuxième terme du signal prétraité est une première fonction f2$_i$ de prédiction inversible qui dépend exclusivement des signaux bruts observés dans le passé proche jusqu'à l'instant $t_{N-p(i)}$, en le nombre entier m(i) d'électrodes voisines $\sigma_i(j)$ de l'électrode observée i et en l'électrode observée i.

Dans ce cas, l'expression du signal prétraité s'écrit :

$$\varepsilon_i(t_N) = u_i(t_N) - f2_i \left( \left( u_{\sigma_i(j)}(t_{N-k}) \right)_{\substack{j \in [1,m(i)] \\ k \in [1,p(i)]}} ; u_i(t_{N-k})_{k \in [1,p(i)]} \right)$$

Equation 2,

dans laquelle :

les influences des échantillons du passés du signal brut de l'électrode i, c'est-à-dire les contributions de ces échantillons passés $u_i(t_{N-k})$, k variant de 1 à p(i) au signal brut observé en l'électrode observée i à l'instant d'échantillonnage courant $t_N$, sont également pris en compte.

Suivant un troisième mode de réalisation du procédé de prétraitement de compression, dérivé du premier mode de réalisation, pour chaque électrode i observée, la fonction de prédiction fi du deuxième terme du signal prétraité est une première fonction de prédiction linéaire f1L$_i$ inversible qui dépend exclusivement des signaux bruts observés dans le passé proche jusqu'à l'instant $t_{N-p(i)}$, en le nombre entier m(i) d'électrodes voisines $\sigma_i(j)$ de l'électrode observée i.

Dans ce cas, l'expression du signal prétraité s'écrit :

$$\varepsilon_i(t_N) = u_i(t_N) - \sum_{k=1}^{p(i)} \sum_{j=1}^{m(i)} a_{ijk} * u_{\sigma_i(j)}(t_{N-k})$$

Equation 3,

dans laquelle les $a_{ijk}$, j variant de 1 à m(i) et k variant de 1 à p(i) sont les coefficient de transformation de la première fonction de prédiction linéaire f1L$_i$.

Suivant un quatrième mode de réalisation du procédé de prétraitement de compression, dérivé du deuxième mode de réalisation, pour chaque électrode i observée, la fonction de prédiction fi du deuxième terme du signal prétraité est une deuxième fonction de prédiction linéaire f2L$_i$ inversible qui dépend des signaux bruts observés dans le passé proche jusqu'à l'instant $t_{N-p(i)}$, en le nombre entier m(i) d'électrodes voisines $\sigma_i(j)$ de l'électrode observée i et en l'électrode observée i.

Dans ce cas, l'expression du signal prétraité s'écrit :

$$\varepsilon_i(t_N) = u_i(t_N) - \sum_{k=1}^{p(i)} \left( a_{i0k} * u_i(t_{N-k}) + \sum_{j=1}^{m(i)} a_{ijk} * u_{\sigma_i(j)}(t_{N-k}) \right)$$

Equation 4,

dans laquelle les $a_{ijk}$, j variant de 1 à m(i) et k variant de 1 à p(i), et les $a_{i0k}$, k variant de 1 à p(i) sont les coefficient de transformation de la deuxième fonction de prédiction linéaire f2L$_i$.

En variante des quatre modes de réalisation du procédé de prétraitement de compression décrit ci-dessus, au moins un des entiers m(i), p(i) est indépendant de l'indice i de l'électrode observée.

En variante, les deux entiers m(i) et p(i) sont indépendants de l'indice i de l'électrode observée i.

Suivant un cinquième mode de réalisation du procédé de prétraitement de compression, dérivé du troisième mode de réalisation, les deux entiers m(i) et p(i) sont indépendants de l'indice i de l'électrode observée i, et pour chaque électrode i observée, la fonction de prédiction fi du deuxième terme du signal prétraité est une première fonction de prédiction linéaire f1 L$_i$ inversible qui dépend exclusivement des signaux bruts observés dans le passé proche jusqu'à l'instant $t_{N-p}$, en le nombre entier fixé m d'électrodes voisines $\sigma_i(j)$ de l'électrode observée i.

Dans ce cas, la transformation réalisée pour le prétraitement des signaux bruts pour chaque voie d'acquisition i d'une électrode observée i se ramène essentiellement à déterminer une combinaison linéaire des voies environnantes pour supprimer la corrélation spatiale et temporelle existant entre des échantillons passés $u_{\sigma_i(j)}(t_{N-k})$ des signaux bruts de ces voies environnantes $\sigma_i(j)$ et le signal brut $u_i(t_N)$ mesuré par la voie d'acquisition i sur l'électrode i observée i correspondante à l'instant d'échantillonnage courant $t_N$

Dans ce cas, l'expression du signal prétraité $\varepsilon_i(t_N)$ s'écrit :

$$\varepsilon_i(t_N) = u_i(t_N) - \sum_{k=1}^{p} \sum_{j=1}^{m} a_{ijk} * u_{\sigma_i(j)}(t_{N-k})$$

Equation 5

Suivant un cas particulier du cinquième mode réalisation, des voies d'acquisition environnantes sont des voies associées à un nombre entier m d'électrodes immédiatement voisines $\sigma_i(j)$ de l'électrode observée i.

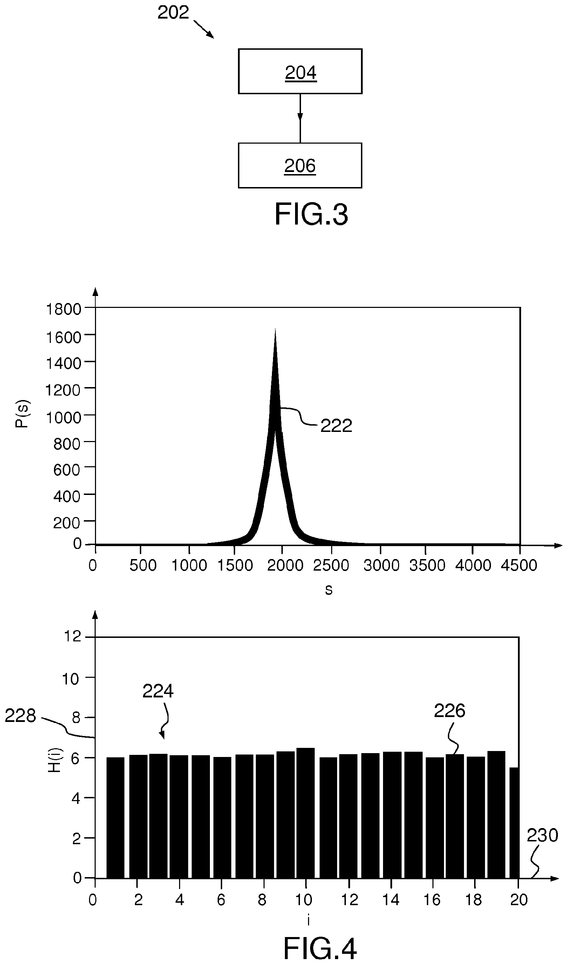

Suivant la Figure 3, un procédé de prétraitement de compression 202 selon l'invention comprend une étape d'apprentissage 204 et une étape de prétraitement de compression effective 206 des données brutes telle que décrite ci-dessus dans les divers modes de réalisation.

L'étape d'apprentissage 204 est exécutée ici en une seule fois pour toute pour l'ensemble des électrodes observées i, i variant de 1 à L, avant l'étape de transformation des données brutes.

Au cours de L'étape d'apprentissage 204, un ensemble de paramètres caractérisant les fonctions de prédiction fi sont déterminés en les ajustant par un traitement statistique, la taille de la statistique étant fonction d'un nombre d'échantillons temporels choisi suffisamment grand pour minimiser les amplitudes des erreurs.

Lorsque les fonctions de prédiction fi sont des fonctions de transformation linéaires, ce sont les coefficients $a_{ijk}$ et/ou $a_{i0k}$ définis dans les équations 3, 4 et 5 qui forment les paramètres des fonctions de prédiction linéaire et forment des matrices de transformations linéaires.

En variante, l'étape d'apprentissage est exécutée de manière étalée dans le temps par paquets d'électrodes et en parallèle de l'étape de transformation 206 des données brutes en des données prétraitées. En d'autres termes, à partir d'un jeu initial de paramètres caractérisant les fonctions de prédictions, les paramètres et au travers d'eux les fonctions de prédiction sont affinés.

Il est à remarquer que le principe d'un procédé selon l'invention d'un prétraitement de compression adapté à des données ECoG est fondé sur l'observation que les données ECoG varient lentement au cours du temps par rapport à la fréquence d'échantillonnage et que les signaux provenant des différentes électrodes dans un voisinage local restreint sont fortement corrélés.

L'efficacité de la compression est évaluée en calculant l'entropie du signal, avant et après la compression. L'entropie reflète le contenu informatif du signal : plus elle est petite, plus le taux de compression obtenu après application d'un encodage entropique sera élevé.

Par exemple, lorsqu'on se limite à un échantillonnage sur 12 bits, l'entropie H d'un signal est définie par l'expression :

$$H = - \sum_{i=1}^{2^{12}} P_s \ln(P_s)$$

Equation 6,

dans laquelle

.- s désigne ici un symbole pris parmi les $2^{12}$ vecteurs de 12 bits possibles du signal ;
.- Ps désigne la probabilité d'occurrence du symbole s dans la série temporelle des échantillons du signal

Suivant le théorème de l'information de Shannon, la valeur de l'entropie donne le nombre minimum de bits sur lequel il faut encoder le signal afin de garder tout son contenu informatif.

Le procédé de prétraitement de compression qui transforme des données brutes d'acquisition de signaux électrocorticographiques sur un ensemble d'électrodes en un jeu de données prétraitées permet d'abaisser l'entropie des données brutes, l'entropie des données de prétraitement compressées étant nettement inférieure à l'entropie des données brutes avant application de la compression.

Il est rappelé que pour retrouver les données d'origine, c'est à dire les données brutes, il suffit de réaliser lors de la décompression de prétraitement l'opération inverse de la transformation appliquée lors du prétraitement de compression car cette transformation est inversible.

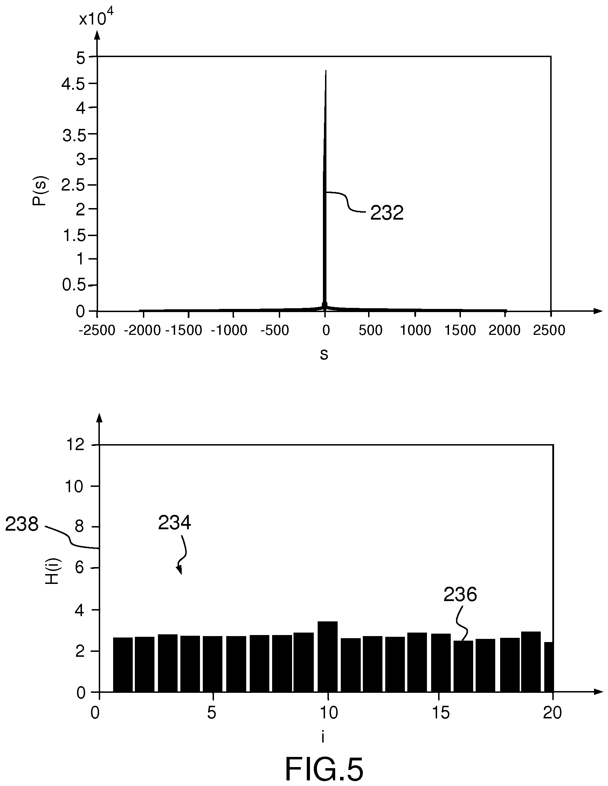

Suivant les Figures 4 et 5, un algorithme de compression selon l'invention a été évalué sur un jeu de données ECoG recueilli sur un singe par une matrice d'électrodes corticales. Ces données correspondent à un enregistrement d'une durée de 250 secondes avec une fréquence d'échantillonnage des signaux observés égale à 1 kHz. Les données ont été importées dans l'outil informatique Matlab puis traitées.

Le traitement appliqué a consisté à mettre les deux étapes 204 et 206 d'un procédé de prétraitement de compression utilisant la fonction de transformation décrite par l'équation 5 avec p et m fixés égaux respectivement à 5 et 2.

Dans la première étape, un apprentissage a d'abord été réalisé sur des données non compressées, c'est-à-dire brutes, et les paramètres des fonctions de prédiction ont été extraits.

Puis, dans la deuxième étape, les paramètres extraits lors de l'étape d'apprentissage ont été utilisés afin de réaliser la compression.

Suivant la vue du haut de la Figure 4, les fonctions de répartition des symboles des 20 signaux brutes acquis par 20 voies d'acquisition et mesurés au travers de 20 électrodes apparaissent sous la forme d'une courbe 222 quasi identique, ce qui montre une uniformité des lois de répartition des symboles sur l'ensemble des voies d'acquisition.

Suivant la vue du bas de la Figure 4, il est représenté sous forme d'un ensemble 224 de bâtonnets 226 l'évolution de l'entropie H(i) de chacune des voies, calculée selon la formule de l'équation 6 et exprimée sur l'axe des ordonnées 228, en fonction de la numérotation des voies d'acquisition par un entier i codé sur l'axe des abscisses 230.

Il est à remarquer que le numéro i de la voie d'acquisition peut être considéré comme l'indice d'identification de l'électrode observée qui lui correspond.

Les entropies H(i) sont sensiblement égales à la valeur de 6,1 qui est égale à la valeur moyenne des entropies H(i) sur l'ensemble des 20 voies d'acquisitions.

Suivant la vue du haut de la Figure 5, les fonctions de répartition des symboles des 20 signaux prétraités correspondant respectivement aux signaux bruts acquis par les 20 voies d'acquisition apparaissent superposés sous la forme d'une courbe 232 quasi identique, ce qui montre une uniformité des lois de répartition des symboles des signaux compressés sur l'ensemble des voies d'acquisition.

La dispersion statistique des symboles sur ces courbes est nettement plus petite que la dispersion statistique des symboles observée sur les courbes de la vue du haut de la Figure 4.

Suivant la vue du bas de la Figure 5, il est représenté sous forme d'un ensemble 234 de bâtonnets 236 l'évolution de l'entropie H(i) de chaque signal compressé correspondant à une voie i, calculée selon la formule de l'équation 6 et codée sur l'axe des ordonnées 238, en fonction du numéro i de la voie d'acquisition représenté sur l'axe des abscisses 240.

Les entropies H(i) des signaux prétraités sont sensiblement égales à la valeur de 2,7 qui est égale à la valeur moyenne des entropies des signaux compressés sur l'ensemble des 20 voies d'acquisitions.

Ainsi l'entropie de l'ensemble des signaux bruts ECoG acquis par l'ensemble des voies d'acquisition a été réduite significativement de 6,1 à 2,7.

Le grand avantage d'un procédé de prétraitement de compression utilisant des fonctions de prédiction linéaires, outre le fait qu'un taux de compression élevé peut être atteint réside dans le fait que les opérations linéaires sont peu complexes à réaliser en termes des fonctions électroniques requises, additions et multiplications, dont le nombre est maîtrisé par l'ordre de l'estimation, fixée au travers des paramètres p et m. On peut ainsi facilement adapter la complexité du prétraitement à la puissance de calcul disponible au sein du compresseur et réaliser une optimisation de la consommation globale de l'unité de transmission en jouant sur le rapport R entre l'énergie de compression $E_{comp}$, dépensée par le compresseur, et l'énergie requise pour la transmission $E_{trans}$ divisée par le taux de compression global, étant entendu que le taux de compression global inclut l'effet de la compression de prétraitement adapté aux signaux ECoG et l'effet d'un encodage entropique générique.

Dans le cas d'une absence de prétraitement de compression de adaptée aux signaux bruts de l'application, c'est-à-dire correspondant au cas des performances entropiques décrite dans la Figure 4, le taux de compression maximal atteignable après application d'un encodage entropique générique est égal à 49%.

Dans le cas de l'utilisation d'un prétraitement de compression adapté aux signaux bruts de l'application, c'est-à-dire correspondant au cas des performances entropiques décrites dans la Figure 5, le taux de compression maximal atteignable après application d'un encodage entropique générique est égal à 77%.

Il est possible de combiner l'approche de prétraitement selon l'invention avec d'autres méthodes, comme une compression avec pertes par analyse de composantes principales, des transformations à ondelettes afin de cumuler les avantages des diverses méthodes de prétraitement, augmenter ainsi le taux de compression, mais au détriment des performances énergétiques.

L'utilisation d'un procédé de l'invention utilisant des fonctions de prédiction peu complexes à réaliser et au final peu énergivores reste privilégiée.

Tous les implants de mesure ECoG nécessitant la transmission de données à haut débit ou le stockage de données peuvent profiter du type de compression proposée selon l'invention car le critère de basse consommation y est primordial.

Seules les erreurs d'estimations $\varepsilon_i(t_N)$ des voies d'acquisition sont transmises de préférence par une liaison sans fil radioélectrique ou stockée avec efficacité dans une mémoire électronique facilement implantable dans un tissu vivant.

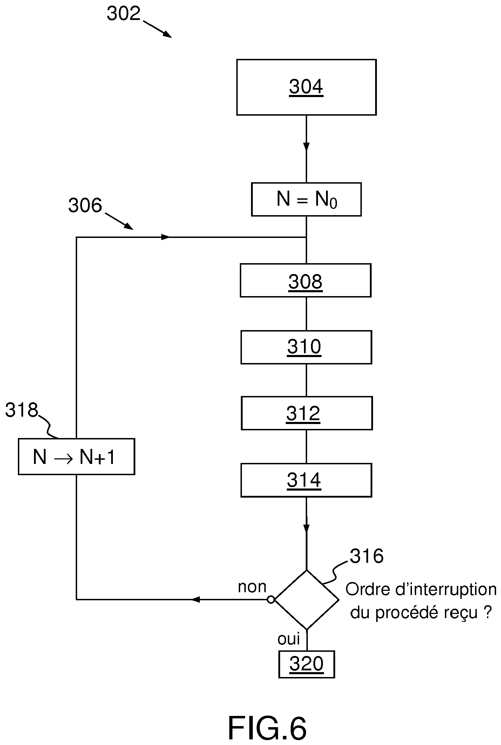

Suivant la Figure 6, un procédé de décompression 302, correspondant au procédé de prétraitement de compression décrit ci-dessus, comprend un ensemble d'étapes.

Dans une première étape d'initialisation 304, un jeu de données d'initialisation pour chaque électrode observée i est transmis au travers d'une première liaison et reçu par le décompresseur de prétraitement au travers du récepteur de transmission.

Les données d'initialisation sont formées par des signaux bruts, observés dans un passé proche jusqu'à l'instant $t_{N0-p}$, p désignant un rang de passé le plus lointain par rapport à un rang $N_0$ de l'instant de début de la mise en œuvre effective de la compression, en un nombre entier m d'électrodes voisines $\sigma_i(j)$ de l'électrode observée i, le jeu de données étant transmis au travers d'une première liaison.

Dans une boucle itérative 306 de parcours d'un rang courant N, pour le rang courant N à partir de $N_0$, et pour chaque électrode i, les étapes suivantes sont exécutées.

Dans une deuxième étape 308 et au préalable, des signaux bruts sont fournis au décompresseur de prétraitement, ces signaux bruts lui ayant été transmis directement au travers de la première liaison ou ayant été reconstruits à l'identique par le décompresseur de prétraitement lui-même à partir de signaux compressés de prétraitement pertinents correspondants, transmis au travers d'une deuxième liaison.

En variante et de manière préférée, la deuxième liaison et la première liaison forment une seule et même liaison.

Les signaux bruts fournis sont les signaux bruts observés dans un passé proche jusqu'à l'instant $t_{N-p}$, p désignant un rang de passé le plus lointain par rapport au rang N de l'instant courant, en le nombre entier m d'électrodes voisines $\sigma_i(j)$ de l'électrode observée ou en le nombre entier m d'électrodes voisines $\sigma_i(j)$ de l'électrode observée i et l'électrode observée i.

Puis, dans une troisième étape 310, la donnée de prétraitement compressée $\varepsilon_i(t_N)$ à l'instant d'échantillonnage $t_N$ correspondant à l'électrode i observée est reçue par le décompresseur de prétraitement, étant rappelé que ladite donnée de prétraitement compressée a été déterminée par la mise en œuvre du procédé de prétraitement de compression décrit ci-dessus et a été transmise au travers de la deuxième liaison.

Ensuite, dans une quatrième étape 312, un deuxième terme de reconstruction est déterminé à partir de la fonction de prédiction fi du deuxième terme, appliquée aux signaux bruts observés dans un passé proche jusqu'à l'instant $t_{N-p}$, en le nombre entier m d'électrodes voisines $\sigma_i(j)$ de l'électrode observée i ou en le nombre entier m d'électrodes voisines $\sigma_i(j)$ de l'électrode observée et l'électrode observée i, les signaux brutes observés étant fournis dans l'étape de fourniture 308.

Dans une cinquième étape 314, la donnée brute du signal acquis à l'instant d'échantillonnage $t_N$ est reconstruite comme étant égale à la somme du deuxième terme de reconstruction et de la donnée prétraitée du signal acquis à l'instant d'échantillonnage $t_N$, en l'électrode i.

Puis après avoir vérifié dans une étape de test 316 qu'aucun ordre d'interrompre le procédé de décompression n'a été reçu, une incrémentation unitaire du rang courant N est exécutée, puis les deuxième, troisième, quatrième, cinquième étapes, 308, 310, 312, 314, et l'étape de test 316 sont répétées.

Dans le cas où un ordre d'interrompre le procédé de décompression a été reçu, le procédé de décompression est interrompu dans une étape d'interruption 320.

Ainsi, le signal d'origine formé par les signaux bruts acquis peut être reconstitué par le décompresseur de prétraitement, réalisé par exemple par un calculateur électronique, en réalisant les transformations inverses des transformations réalisées au cours de la compression de prétraitement et en y ajoutant les erreurs d'estimation reçues formant les données compressées transmises.

Ici, la première liaison est une liaison filaire et la deuxième liaison est une liaison sans fil radioélectrique.

En variante et de manière préférée, comme représenté sur la Figure 1, les première et deuxième liaisons forment une unique liaison radioélectrique de transmission de données.

Le système d'acquisition et de transmission de données de la Figure 1 comporte un ou plusieurs calculateurs électroniques dans lesquels un ensemble d'instructions formant un programme d'ordinateur sont chargées et exécutées afin de mettre en œuvre le procédé de compression et/ou le procédé de décompression définis ci-dessus.

## Revendications

1. Procédé de compression de données brutes $u_i(t_N)$ de mesures de signaux électrocorticographiques évoluant dans le temps et acquises à l'aide d'un ensemble (4) d'électrodes (6) disposées en contact direct d'un cortex (24),

   chaque électrode étant **caractérisée par** un indice entier i différent d'identification et une position r(i) à la surface du cortex (24), l'indice i variant de 1 à L et L désignant le nombre total des électrodes (6),

   le signal temporel électrocorticographique brut $u_i(t)$ acquis par l'électrode d'indice i étant échantillonné à des instants successifs $t_N$, N étant un entier désignant le rang de succession d'un échantillon du signal dans le temps, en un signal brut échantillonné $u_i(t_N)$ dont le codage de l'amplitude forme les données brutes du signal brut électrocorticographique acquis à l'instant d'échantillonnage $t_N$,

   le procédé de compression étant **caractérisé en ce qu'**il comprend une étape effective de prétraitement de compression du signal temporel électrocorticographique brut échantillonné $u_i(t_N)$ avant un codage entropique consistant à :

   pour i variant de 1 à L et à chaque instant d'échantillonnage successif $t_N$ à partir d'un rang $N_0$, transformer le signal brut acquis $u_i(t_N)$ par l'électrode i observée en un signal prétraité $\varepsilon_i(t_N)$ égal à la différence entre un premier terme et un deuxième terme,

   le premier terme étant égal au signal brut $u_i(t_N)$ acquis en l'électrode i à l'instant courant $t_N$, et

   le deuxième terme étant une fonction fi de prédiction, sans pertes d'information et calculable en temps réel, qui dépend temporellement de signaux bruts $u_{\sigma_i(j)}(t_{N-k})$, $u_i(t_{N-k})$ observés dans un passé proche jusqu'à l'instant $t_{N-p(i)}$, p(i) désignant un rang entier non nul de passé le plus lointain par rapport au rang N de l'instant courant associé à l'électrode observée i, k étant un entier variant de 1 à p(i), en au moins un nombre entier m(i), différent de L, d'électrodes voisines $\sigma_i(j)$ de l'électrode observée i et au plus en le nombre entier m(i) d'électrodes voisines $\sigma_i(j)$ de l'électrode observée i et en l'électrode observée i, $\sigma_i$ étant une fonction de voisinage de l'électrode i observée et $\sigma_i(j)$ désignant les indices des m(i) électrodes voisines de l'électrode i observée, j étant un indice de parcours de la fonction de voisinage $\sigma_i$ variant de 1 à m(i).

2. Procédé de compression selon la revendication 1, dans lequel la fonction fi du deuxième terme est une première fonction $f1_i$ de prédiction qui dépend exclusivement des signaux bruts observés $u_{\sigma_i(j)}(t_{N-k})$ dans le passé proche jusqu'à l'instant $t_{N-p(i)}$, en le nombre entier m(i) d'électrodes voisines $\sigma_i(j)$ de l'électrode observée i, $\sigma_i$ étant une fonction de voisinage de l'électrode i observée.

3. Procédé de compression selon la revendication 1, dans lequel la fonction fi du deuxième terme est une deuxième fonction $f2_i$ de prédiction qui dépend exclusivement des signaux bruts $u_{\sigma_i(j)}(t_{N-k})$, $u_i(t_{N-k})$, observés dans le passé proche jusqu'à l'instant $t_{N-p(i)}$, en le nombre entier m(i) d'électrodes voisines $\sigma_i(j)$ de l'électrode observée i et en l'électrode observée i.

4. Procédé de compression selon l'une quelconque des revendications 1 à 3, dans lequel la fonction fi est une fonction de prédiction linéaire $f1L_i$ qui dépend exclusivement des signaux bruts observés dans le passé proche jusqu'à l'instant $t_{N-p(i)}$, en le nombre entier m(i) d'électrodes voisines $\sigma_i(j)$ de l'électrode observée i ou une fonction linéaire f2L$_i$ qui dépend exclusivement des signaux bruts observés dans le passé proche jusqu'à l'instant $t_{N-p(i)}$, en le nombre entier m(i) d'électrodes voisines $\sigma_i(j)$ de l'électrode observée i et en l'électrode observée i.

5. Procédé de compression selon l'une quelconque des revendications 1 à 4 dans lequel
au moins un des entiers m(i), p(i) est dépendant de l'indice i de l'électrode observée, ou
les deux entiers m(i) et p(i) sont dépendants de l'indice i de l'électrode observée.

6. Procédé de compression selon l'une quelconque des revendications 1 à 5, dans lequel le deuxième terme est une fonction de prédiction qui dépend de signaux bruts observés dans un passé proche jusqu'à l'instant $t_{N-p(i)}$, en un nombre entier m(i) d'électrodes immédiatement voisines $\sigma_i(j)$ de l'électrode observée i.

7. Procédé de compression selon l'une quelconque des revendications 1 à 6, comprenant une étape d'apprentissage (206), exécutée soit une seule fois pour toute pour l'ensemble des électrodes observées i, i variant de 1 à L, avant l'étape effective (206) de prétraitement de compression des données brutes, soit de manière étalée dans le temps par paquet d'électrodes et en parallèle de l'étape effective (206) de prétraitement de compression des données brutes en des données prétraitées, et au cours de laquelle un ensemble de paramètres caractérisant les fonctions de prédiction fi sont déterminés en les ajustant par un traitement statistique, la taille de la statistique étant fonction d'un nombre d'échantillons temporels suffisamment grand.

8. Procédé de compression selon les revendications 4 et 7, dans lequel les paramètres des fonctions de prédiction linéaires sont des matrices de coefficients de transformations linéaires.

9. Procédé de décompression correspondant au procédé de compression défini selon l'une quelconque des revendications 1 à 8, comprenant les étapes consistant à :

.- dans une première étape d'initialisation (304), recevoir pour chaque électrode observée i un jeu de données d'initialisation formées par des signaux brutes observés dans un passé proche jusqu'à l'instant $t_{N0-p(i)}$, p(i) désignant un rang de passé le plus lointain par rapport à un rang $N_0$ de l'instant de début de la mise en œuvre effective de la compression, en un nombre entier m(i) d'électrodes voisines $\sigma_i(j)$ de l'électrode observée i, le jeu de données étant transmis au travers d'une première liaison ; puis
dans une boucle itérative (306) de parcours d'un rang courant N, pour le rang courant N à partir de $N_0$, et pour chaque électrode i,
.- dans une deuxième étape (308), fournir des signaux brutes observés dans un passé proche jusqu'à l'instant $t_{N-p(i)}$, p(i) désignant un rang de passé le plus lointain par rapport au rang N de l'instant courant, en le nombre entier m(i) d'électrodes voisines $\sigma_i(j)$ de l'électrode observée ou en le nombre entier m(i) d'électrodes voisines $\sigma_i(j)$ de l'électrode observée i et l'électrode observée i, transmis directement au travers de la première liaison ou reconstruits à partir de signaux compressés pertinents correspondants transmis au travers d'une deuxième liaison; puis
.- dans une troisième étape (310), recevoir la donnée prétraitée $\varepsilon_i(t_N)$ du signal acquis à l'instant d'échantillonnage $t_N$, en l'électrode i, ladite donnée prétraitée ayant été déterminée par la mise en œuvre du procédé de prétraitement défini selon l'une des revendications 1 à 8, et ayant été transmise au travers d'une deuxième liaison ; puis
.- dans une quatrième étape (312), déterminer un deuxième terme de reconstruction à partir de la fonction de prédiction fi du deuxième terme, appliquée aux signaux bruts observés dans un passé proche jusqu'à l'instant $t_{N-p(i)}$, en le nombre entier m(i) d'électrodes voisines $\sigma_i(j)$ de l'électrode observée i ou en le nombre entier m(i) d'électrodes voisines $\sigma_i(j)$ de l'électrode observée i et l'électrode observée i, les signaux brutes observés étant fournis au préalable dans la deuxième étape de fourniture (308), puis
.- dans une cinquième étape (314), reconstruire la donnée brute $u_i(t_N)$ du signal acquis à l'instant d'échantillonnage $t_N$, comme étant égal à la somme du deuxième terme de reconstruction et de la donnée prétraitée $\varepsilon_i(t_N)$ du signal acquis à l'instant d'échantillonnage $t_N$, en l'électrode i.

10. Procédé de décompression selon la revendication 9 dans lequel,
les première et deuxième liaisons sont la même liaison, ou
la première liaison est une liaison filaire et la deuxième liaison est une liaison sans fil.

11. Unité de transmission de données de mesure de signaux électrocorticographiques évoluant dans le temps comprenant une unité de prétraitement de compression (28) de données pour convertir des données brutes $u_i(t_N)$ de mesures de signaux électrocorticographiques prélevés par des électrodes (6) en divers emplacements à la surface d'un cortex (24) en des données de prétraitement compressées $\varepsilon_i(t_N)$, et un émetteur de transmission (12) des données compressées sur une liaison,

**caractérisée en ce que** l'unité de prétraitement de compression (28) est configuré pour mettre en œuvre l'étape effective de prétraitement de compression défini selon l'une quelconque des revendications 1 à 8.

**12.** Unité de transmission de données selon la revendication 11, dans laquelle l'unité de prétraitement de compression (28) et l'émetteur de transmission (12) sont miniaturisés pour être implantés dans le corps d'un patient et l'émetteur de transmission (12) est configuré pour émettre des ondes radioélectriques.

**13.** Système d'acquisition et de transmission de données de mesures de signaux électrocorticographiques évoluant dans le temps comprenant

.- un ensemble (4) d'électrodes (6) disposées en contact direct d'un cortex (24), caractérisées chacune par un indice entier i différent d'identification et une position r(i) à la surface du cortex, l'indice i variant de 1 à L et L désignant le nombre total d'électrodes (6), et configurées chacune pour acquérir un signal temporel électrocorticographique brute $u_i(t)$ ; et

.- une unité d'échantillonnage (8), configurée pour échantillonner à des instants successifs $t_N$, N étant un entier désignant le rang de succession d'un échantillon d'un signal dans le temps, les signaux temporels électrocorticographiques brutes $u_i(t)$ et coder leur amplitude en des données brutes associés ;

.- une unité de prétraitement de compression (28) de données pour convertir les données brutes $u_i(t_N)$ en des données prétraitées $\varepsilon_i(t_N)$, à entropie moins élevée ;

.- un encodeur entropique (30) ; et

.- un émetteur de transmission (12) pour transmettre les données prétraitées compressées ;

le système d'acquisition et de transmission étant **caractérisé en ce que** l'unité de prétraitement de compression (28) est configurée pour mettre en œuvre une étape effective ((204) de prétraitement de compression des données brutes $u_i(t_N)$ consistant à : pour i variant de 1 à L et à chaque instant d'échantillonnage successifs $t_N$ à partir d'un rang $N_0$, transformer le signal brut $u_i(t_N)$ acquis par l'électrode i en un signal de prétraité $\varepsilon_i(t_N)$ égal à la différence entre un premier terme et un deuxième terme, le premier terme étant égal au signal brut $u_i(t_N)$ acquis en l'électrode i à l'instant courant $t_N$, et

le deuxième terme étant une fonction fi de prédiction, sans pertes d'information et calculable en temps réel, qui dépend temporellement de signaux bruts $u_{\sigma_i(j)}(t_{N-k})$, $u_i(t_{N-k})$ observés dans un passé proche jusqu'à l'instant $t_{N-p(i)}$, p(i) désignant un rang entier non nul de passé le plus lointain par rapport au rang N de l'instant courant associé à l'électrode observée i, k étant un entier variant de 1 à p(i), en au moins un nombre entier m(i), différent de L, d'électrodes voisines $\sigma_i(j)$ de l'électrode observée i et au plus en le nombre entier m(i) d'électrodes voisines $\sigma_i(j)$ de l'électrode observée et en l'électrode observée i, $\sigma_i$ étant une fonction de voisinage de l'électrode i observée et $\sigma_i(j)$ désignant les indices des m(i) électrodes voisines de l'électrode i observée, j étant un indice de parcours de la fonction de voisinage $\sigma_i$ variant de 1 à m(i).

**14.** Système d'acquisition et de transmission de données de mesures de signaux électrocorticographiques selon la revendication 13, comprenant en outre :

.- un récepteur de transmission (14), distant de l'émetteur de transmission (12), pour recevoir les données compressées,

.- un décodeur entropique (44), et

.- un décompresseur de prétraitement (46) pour convertir les données prétraitées $\varepsilon_i(t_N)$ en les données brutes $u_i(t_N)$, configuré pour exécuter les étapes définies selon l'une quelconque des revendications 9 et 10.

**15.** Produit ou programme d'ordinateur comprenant un ensemble d'instructions configurées pour mettre en œuvre le procédé de compression défini selon l'une quelconque des revendications 1 à 8 et/ou le procédé de décompression défini selon l'une quelconque des revendications 9 et 10 lorsqu'elles sont chargées dans et exécutées par un calculateur formant le compresseur et/ou par un calculateur formant le décompresseur défini selon l'une quelconque des revendications 13 à 14.

**Patentansprüche**

**1.** Verfahren zum Komprimieren von Rohdaten $u_i(t_N)$ der Messungen von elektrokortikografischen Signalen, die sich im Laufe der Zeit verändern und mittels einer Gruppe (4) von Elektroden (6) erfasst werden, die in direktem Kontakt mit einem Kortex (24) angeordnet sind,
wobei jede Elektrode durch einen anderen ganzzahligen Identifikationsindex i und eine Position r(i) auf der Oberfläche des Kortex (24) gekennzeichnet ist, wobei der Index i von 1 bis L variiert und wobei L die Gesamtzahl der Elektroden (6) bezeichnet,
wobei das von der Elektrode mit Index i erfasste rohe elektrokortikografische Zeitsignal $u_i$(t) zu aufeinander folgenden Zeitpunkten $t_N$, wobei N eine ganze Zahl ist, die den Rang der zeitlichen Abfolge einer Abtastung des Signals bezeichnet, in ein abgetastetes Rohsignal $u_i(t_N)$ abgetastet wird, dessen Codierung der Amplitude die Rohdaten des zum Abtastzeitpunkt $t_N$ erfassten elektrokortikografischen Rohsignals bildet,
wobei das Kompressionsverfahren **dadurch gekennzeichnet ist, dass** es einen effektiven Schritt der Kompressionsvorbehandlung des abgetasteten rohen elektrokortikografischen Zeitsignals $u_i(t_N)$ vor einer Entropiecodierung beinhaltet, bestehend aus:

Transformieren, für i, das von 1 bis L variiert, und zu jedem nachfolgenden Abtastzeitpunkt $t_N$ ab einem Rangs $N_0$, des von der beobachteten Elektrode i erfassten Rohsignals $u_i(t_N)$ in ein vorbehandeltes Signal $\varepsilon_i(t_N)$ gleich der Differenz zwischen einem ersten Term und einem zweiten Term,
wobei der erste Term gleich dem an der Elektrode i zum aktuellen Zeitpunkt $t_N$ erfassten Rohsignal $u_i(t_N)$ ist, und wobei der zweite Term eine Vorhersagefunktion fi ohne Informationsverlust und in Echtzeit berechenbar ist, die zeitlich von Rohsignalen $u_{\sigma_i(j)}(t_{N-k})$, $u_i(t_{N-k})$ abhängig ist, die in der nahen Vergangenheit bis zum Zeitpunkt $t_{N-p}(i)$ beobachtet wurden, wobei p(i) einen ganzzahligen Rang ungleich null der am weitesten zurückliegenden Vergangenheit in Bezug auf Rang N des mit der beobachteten Elektrode i assoziierten aktuellen Zeitpunkts designiert, wobei k eine ganze Zahl ist, die von 1 bis p(i) variiert, in mindestens einer ganzen Zahl m(i), die sich von L unterscheidet, von der beobachteten Elektrode i benachbarten Elektroden $\sigma_i(j)$, und höchstens in der ganzen Zahl m(i) von der beobachteten Elektrode i benachbarten Elektroden $\sigma_i(j)$, und in der beobachteten Elektrode i, wobei $\sigma_i$ eine Nachbarfunktion der beobachteten Elektrode i ist, und wobei $\sigma_i(j)$ die Indexe der der beobachteten Elektroden i benachbarten m(i) Elektroden designieren, wobei j ein Durchlaufindex der Nachbarfunktion $\sigma_i$ ist, die von 1 bis m(i) variiert.

2. Kompressionsverfahren nach Anspruch 1, bei dem die Funktion fi des zweiten Terms eine erste Vorhersagefunktion $f1_i$ ist, die ausschließlich von den in der nahen Vergangenheit bis zum Zeitpunkt $t_{N-p(i)}$ beobachteten Rohsignalen $u_{\sigma_i(j)}(t_{N-k})$ abhängig ist, in der ganzen Zahl m(i) von der beobachteten Elektrode i benachbarten Elektroden $\sigma_i(j)$, wobei $\sigma_i$ eine Nachbarfunktion der beobachteten Elektrode i ist.

3. Kompressionsverfahren nach Anspruch 1, bei dem die Funktion fi des zweiten Terms eine zweite Vorhersagefunktion $f2_i$ ist, die ausschließlich von den in der nahen Vergangenheit bis zum Zeitpunkt $t_{N-p(i)}$ beobachteten Rohsignalen $u_{\sigma_i(j)}(t_{N-k})$, $u_i(t_{N-k})$ abhängig ist, in der ganzen Zahl m(i) von der beobachteten Elektrode i benachbarten Elektroden $\sigma_i(j)$ und in der beobachteten Elektrode i.

4. Kompressionsverfahren nach einem der Ansprüche 1 bis 3, bei dem die Funktion fi eine lineare Vorhersagefunktion $f1L_i$ ist, die ausschließlich von den in der nahen Vergangenheit bis zum Zeitpunkt $t_{N-p(i)}$ beobachteten Rohsignalen abhängig ist, in der ganzen Zahl m(i) von der beobachteten Elektrode i benachbarten Elektroden $\sigma_i(j)$, oder eine lineare Funktion $f2L_i$, die ausschließlich von den in der nahen Vergangenheit bis zum Zeitpunkt $t_{N-p(i)}$ beobachteten Rohsignalen abhängig ist, in der ganzen Zahl m(i) von der beobachteten Elektrode i benachbarten Elektroden $\sigma_i(j)$ und in der beobachteten Elektrode i.

5. Kompressionsverfahren nach einem der Ansprüche 1 bis 4, bei dem
mindestens eine der ganzen Zahlen m(i), p(i) vom Index i der beobachteten Elektrode abhängig ist, oder
die beiden ganzen Zahlen m(i) und p(i) vom Index i der beobachteten Elektrode abhängig sind.

6. Kompressionsverfahren nach einem der Ansprüche 1 bis 5, bei dem der zweite Term eine Vorhersagefunktion ist, die von in der nahen Vergangenheit bis zum Zeitpunkt $t_{N-p(i)}$ beobachteten Rohsignalen abhängig ist, in einer ganzen Zahl m(i) von der beobachteten Elektrode i unmittelbar benachbarten Elektroden $\sigma_i(j)$.

7. Kompressionsverfahren nach einem der Ansprüche 1 bis 6, das einen Lernschritt (206) beinhaltet, ausgeführt entweder ein einziges Mal für den gesamten Satz von beobachteten Elektroden i, wobei i von 1 bis L variiert, vor dem effektiven Schritt (206) des Kompressionsvorbehandelns der Rohdaten, oder auf zeitlich verteilte Weise per Elektrodenpaket und parallel zu dem effektiven Schritt (206) der Kompressionsvorbehandlung der Rohdaten in vorbehandelte Daten, und in dessen Verlauf ein Satz von Parametern, der die Vorhersagefunktion fi charakterisiert, durch Justieren derselben durch eine statistische Behandlung bestimmt werden, wobei die Größe der Statistik von einer ausreichend großen Anzahl von zeitlichen Samples abhängig ist.

8. Kompressionsverfahren nach den Ansprüchen 4 und 7, wobei die Parameter der linearen Vorhersagefunktion lineare Transformationskoeffizientenmatrizen sind.

9. Dekompressionsverfahren entsprechend dem Kompressionsverfahren wie in einem der Ansprüche 1 bis 8 definiert, das die folgenden Schritte beinhaltet:

- Empfangen für jede beobachtete Elektrode i, in einem ersten Initialisierungsschritt (304), einer Reihe von Initialisierungsdaten, gebildet durch in der nahen Vergangenheit bis zum Zeitpunkt $t_{N0-p(i)}$ beobachteten Rohsignale, wobei p(i) einen Rang der am weitesten zurückliegenden Vergangenheit in Bezug auf einen Rang $N_0$ des Anfangszeitpunkts der effektiven Durchführung der Kompression designiert, in einer ganzen Zahl m(i) von der beobachteten Elektrode i benachbarten Elektroden $\sigma_i(j)$, wobei die Datenreihe über eine erste Verbindung übertragen wird; dann

in einer iterativen Durchlaufschleife (306) eines aktuellen Rangs N, für den aktuellen Rang N ab $N_0$, und für jede Elektrode i:

- Liefern, in einem zweiten Schritt (308), der in einer nahen Vergangenheit bis zum Zeitpunkt $t_{N-p(i)}$ beobachteten Rohsignale, wobei p(i) einen Rang der am weitesten zurückliegenden Vergangenheit in Bezug auf den Rang N des aktuellen Zeitpunkts designiert, in der ganzen Zahl m(i) von der beobachteten Elektrode benachbarten Elektroden $\sigma_i(j)$ oder in der ganzen Zahl m(i) von der beobachteten Elektrode i benachbarten Elektroden $\sigma_i(j)$ und der beobachteten Elektrode i, die direkt über die erste Verbindung übertragen werden oder auf der Basis von über eine zweite Verbindung übertragenen entsprechenden relevanten komprimierten Signalen rekonstruiert werden; dann
- Empfangen, in einem dritten Schritt (310), der vorbehandelten Daten $\varepsilon_i(t_N)$ des zum Abtastzeitpunkt $t_N$ erfassten Signals, in der Elektrode i, wobei die vorbehandelten Daten durch Durchführen des in einem der Ansprüche 1 bis 8 definierten Vorbehandlungsverfahrens bestimmt wurden und über eine zweite Verbindung übertragen wurden; dann
- Bestimmen, in einem vierten Schritt (312), eines zweiten Rekonstruktionsterms auf der Basis der Vorhersagefunktion fi des zweiten Terms, angewendet auf die in einer nahen Vergangenheit bis zum Zeitpunkt $t_{N-p(i)}$ beobachteten Rohsignale, in der ganzen Zahl m(i) von der beobachteten Elektrode i benachbarten Elektroden $\sigma_i(j)$ oder in der ganzen Zahl m(i) von der beoabachten Elektrode i benachbarten Elektroden $\sigma_i(j)$ und der beobachteten Elektrode i, wobei die beobachteten Rohsignale vorab im zweiten Lieferschritt (308) geliefert werden, dann
- Rekonstruieren, in einem fünften Schritt (314), der Rohdaten $u_i(t_N)$ des zum Abtastzeitpunkt $t_N$ erfassten Signals als gleich der Summe des zweiten Rekonstruktionsterms und der vorbehandelten Daten $\varepsilon_i(t_N)$ des zum Abtastzeitpunkt $t_N$ erfassten Signals, in der Elektrode i.

10. Dekompressionsverfahren nach Anspruch 9, bei dem
die erste und zweite Verbindung dieselbe Verbindung sind, oder
die erste Verbindung eine Drahtverbindung und die zweite Verbindung eine drahtlose Verbindung ist.

11. Einheit zum Übertragen von Daten der Messungen von elektrokortikografischen Signalen, die sich im Laufe der Zeit verändern, umfassend eine Datenkompressionsvorbehandlungseinheit (28) zum Konvertieren der von den Elektroden (6) an diversen Stellen auf der Oberfläche eines Kortex (24) entnommenen Rohdaten $u_i(t_N)$ der Messungen von elektrokortikografischen Signalen in komprimierte Vorbehandlungsdaten $\varepsilon_i(t_N)$ und einen Sender (12) zum Übertragen der komprimierten Daten auf einer Verbindung,
dadurch gekennzeichnet, dass die Kompressionsvorbehandlungseinheit (28) zum Durchführen des wie in einem der Ansprüche 1 bis 8 definierten effektiven Schritts der Kompressionsvorbehandlung konfiguriert ist.

12. Datenübertragungseinheit nach Anspruch 11, bei der die Kompressionsvorbehandlungseinheit (28) und der Übertragungssender (12) miniaturisiert sind, um in den Körper eines Patienten implantiert zu werden, und der Übertragungssender (12) zum Aussenden von radioelektrischen Wellen konfiguriert ist.

13. System zum Erfassen und Übertragen von elektrokortikografischen Signalmessdaten, die sich im Laufe der Zeit verändern, das Folgendes umfasst:

- eine Gruppe (4) von Elektroden (6), die in direktem Kontakt mit einem Kortex (24) angeordnet sind, jeweils **gekennzeichnet durch** einen anderen ganzzahligen Identifikationsindex i und eine Position r(i) auf der Oberfläche des Kortex, wobei der Index i von 1 bis L variiert und wobei L die Gesamtzahl von Elektroden (6) bezeichnet, und jeweils konfiguriert zum Erfassen eines rohen elektrokortikografischen Zeitsignals $u_i(t)$; und
- eine Abtasteinheit (8), konfiguriert zum Abtasten zu aufeinander folgenden Zeitpunkten $t_N$, wobei N eine ganze Zahl ist, die den Rang der Abfolge einer Signalabtastung im Laufe der Zeit designiert, der rohen elektrokortikografischen Zeitsignale $u_i(t)$, und Codieren ihrer Amplitude in assoziierte Rohdaten;
- eine Datenkompressionsvorbehandlungseinheit (28) zum Umwandeln der Rohdaten $u_i(t_N)$ in vorbehandelte Daten $\varepsilon_i(t_N)$ mit weniger erhöhter Entropie;
- einen Entropie-Encoder (30); und
- einen Übertragungssender (12) zum Übertragen der komprimierten vorbehandelten Daten;

wobei das Erfassungs- und Übertragungssystem **dadurch** gekennzeichnet ist, dass die Kompressionsvorbehandlungseinheit (28) zum Durchführen eines effektiven Schrittes (204) der Kompressionsvorbehandlung von rohen Daten $u_i(t_N)$ konfiguriert ist, bestehend aus: wenn i von 1 bis L variiert und zu jedem aufeinander folgenden Abtastzeitpunkt $t_N$ ab einem Rang $N_0$, Umwandeln des von der Elektrode i erfassten Rohsignals $u_i(t_N)$ in ein vorbehandeltes Signal $\varepsilon_i(t_N)$ gleich der Differenz zwischen einem ersten Term und einem zweiten Term, wobei der erste Term gleich dem in der Elektrode i zum aktuellen Zeitpunkt $t_N$ erfassten Rohsignal $u_i(t_N)$ ist, und

wobei der zweite Term eine Vorhersagefunktion fi ist, ohne Informationsverlust und in Echtzeit berechenbar, die zeitlich von in der nahen Vergangenheit bis zum Zeitpunkt $t_{N-p(i)}$ beobachteten Rohsignalen $u_{\sigma_i}(t_{N-k})$, $u_i(t_{N-k})$ abhängig ist, wobei p(i) einen ganzzahligen Rang ungleich null der am weitesten zurückliegenden Vergangenheit in Bezug auf den Rang N des mit der beobachteten Elektrode i assoziierten aktuellen Zeitpunkts bezeichnet, wobei k eine ganze Zahl ist, die von 1 bis p(i) variiert, in mindestens einer ganzen Zahl m(i), die sich von L unterscheidet, von der beobachteten Elektrode i benachbarten Elektroden $\sigma_i(j)$, und höchstens der ganzen Zahl m(i) von der beobachteten Elektrode benachbarten Elektroden $\sigma_i(j)$ und in der beobachteten Elektrode i, wobei $\sigma_i$ eine Nachbarfunktion der beobachteten Elektrode i ist und wobei $\sigma_i(j)$ die Indexe der m(i) der beobachteten Elektrode i benachbarten Elektroden bezeichnet, wobei j ein Durchlaufindex der Nachbarfunktion $\sigma_i$ ist, die von 1 bis m(i) variiert.

14. System zum Erfassen und Übertragen von elektrokortikografischen Signalmessdaten nach Anspruch 13, das ferner Folgendes umfasst:

- einen Übertragungsempfänger (14), der vom Übertragungssender (12) entfernt ist, um die komprimierten Daten zu empfangen,
- einen Entropie-Decoder (44), und
- einen Vorbehandlungsdekomprimierer (46) zum Umwandeln der vorbehandelten Daten $\varepsilon_i(t_N)$ in die Rohdaten $u_i(t_N)$, konfiguriert zum Ausführen der in einem der Ansprüche 9 und 10 definierten Schritte.

15. Computerprodukt oder -programm, das einen Satz von Befehlen umfasst, konfiguriert zum Durchführen des in einem der Ansprüche 1 bis 8 definierten Kompressionsverfahrens und/oder des in einem der Ansprüche 9 und 10 definierten Dekompressionsverfahrens, wenn sie in einen Rechner, der den Kompressor bildet, geladen und dort ausgeführt werden und/oder durch einen Rechner, der den in einem der Ansprüche 13 bis 14 definierten Dekomprimierer bildet.

**Claims**

1. Compression method for raw data $u_1(t_N)$ of measurements of electrocorticographic signals which evolve over time and are acquired using a set (4) of electrodes (6) arranged in direct contact with a cortex (24),
each electrode being **characterised by** a different whole identification index i and a position r(i) at the surface of the cortex (24), the index i varying from 1 to L and L denoting the total number of electrodes (6),
the raw electrocorticographic time signal $u_i(t)$ acquired by the electrode having the index i being sampled at successive times $t_N$, N being a whole number denoting the succession rank of a sample of the signal over time, as a sampled raw signal $u_i(t_N)$ whose amplitude encoding forms the raw data of the raw electrocorticographic signal acquired at the sampling time $t_N$,
the compression method being **characterised in that** it comprises an effective step of compression pre-processing of the sampled raw electrocorticographic time signal $u_i(t_N)$ before entropic encoding involving:

for i varying from 1 to L and at each successive sampling time tN from a rank $N_0$, converting the raw signal $u_i(t_N)$ acquired via the electrode i observed into a pre-processed signal $\varepsilon_i(t_N)$ equal to the difference between a first term and a second term,

the first term being equal to the raw signal $u_i(t_N)$ acquired at the electrode i at the current time tN, and

the second term being a prediction function fi which has no loss of information and which can be calculated in real time and which is temporally dependent on raw signals $u_{\sigma_i(j)}(t_{N-k})$, $u_i(t_{N-k})$ observed in the recent past up to the time $t_{N-p(i)}$, p(i) designating an entire rank which is not equal to zero of the most distant past relative to the rank N of the current time associated with the observed electrode i, k being a whole number varying from 1 to p(i), in at least one whole number m(i), different from L, of electrodes $\sigma_i(j)$ adjacent to the observed electrode i and at most in the whole number m(i) of electrodes $\sigma_i(j)$ adjacent to the observed electrode i and in the observed electrode i, $\sigma_i$ being a proximity function of the observed electrode i and $\sigma_i(j)$ denoting the indexes of the m(i) electrodes adjacent to the observed electrode i, j being a travel index of the proximity function $\sigma_i$ varying from 1 to m(i).

2. Compression method according to claim 1, wherein the function fi of the second term is a first prediction function $f1_i$ which is dependent exclusively on the raw signals $u_{\sigma_i(j)}(t_{N-k})$ observed in the recent past up to the time $t_{N-p(i)}$, in the whole number m(i) of electrodes $\sigma_i(j)$ adjacent to the observed electrode i, $\sigma_i$ being a proximity function of the observed electrode i.

3. Compression method according to claim 1, wherein the function fi of the second term is a second prediction function $f2_i$ which is dependent exclusively on the raw signals $u_{\sigma_i(j)}(t_{N-k})$, $u_i(t_{N-k})$ observed in the recent past up to the time $t_{N-p(i)}$, in the whole number m(i) of electrodes $\sigma_i(j)$ adjacent to the observed electrode i and in the observed electrode i.

4. Compression method according to any one of claims 1 to 3, wherein the function fi is a linear prediction function $f1L_i$ which is dependent exclusively on the raw signals observed in the recent past up to the time $t_{N-p(i)}$, in the whole number m(i) of electrodes $\sigma_i(j)$ adjacent to the observed electrode I, or a linear function f2Li which is dependent exclusively on the raw signals observed in the recent past up to the time $t_{N-p(i)}$, in the whole number m(i) of electrodes $\sigma_i(j)$ adjacent to the observed electrode i and in the observed electrode i.

5. Compression method according to any one of claims 1 to 4, wherein

at least one of the whole numbers m(i), p(i) is dependent on the index i of the electrode observed, or

the two whole numbers m(i) and p(i) are dependent on the index i of the electrode observed.

6. Compression method according to any one of claims 1 to 5, wherein the second term is a prediction function which is dependent on raw signals observed in the recent past up to the time $t_{N-p(i)}$, in a whole number m(i) of electrodes $\sigma_i(j)$ immediately adjacent to the observed electrode i.

7. Compression method according to any one of claims 1 to 6, comprising a learning step (206) which is carried out either only once for the entire set of the observed electrodes i, i varying from 1 to L, before the effective step (206) for compression pre-processing of the raw data, or in a manner staggered over time per packet of electrodes and in parallel with the effective step (206) for compression pre-processing of the raw data in pre-processed data, and during which a set of parameters which characterise the prediction functions fi are determined by being adjusted using a statistical processing, the size of the statistic being a function of a sufficiently large number of time samples.

8. Compression method according to claims 4 and 7, wherein the parameters of the linear prediction functions are linear transformation coefficient matrices.

9. Decompression method corresponding to the compression method defined according to any one of claims 1 to 8, comprising the steps involving:

- in a first initialisation step (304), receiving for each observed electrode i a set of initialisation data formed by raw signals observed in the recent past up to the time $t_{N0-p(i)}$, p(i) denoting a rank of the most distant past relative to a rank $N_0$ of the start time of the effective implementation of the compression, in a whole number m(i) of electrodes $\sigma_i(j)$ adjacent to the observed electrode i, the set of data being transmitted via a first connection; then in an iterative travel loop (306) of a current rank N, for the current rank N from $N_0$, and for each electrode i,

- in a second step (308), providing raw signals observed in the recent past up to the time $t_{N-p(i)}$, p(i) denoting a rank of the most distant past relative to the rank N of the current time, in the whole number m(i) of electrodes $\sigma_i(j)$ adjacent to the observed electrode, or in the whole number m(i) of electrodes $\sigma_i(j)$ adjacent to the observed electrode i and the observed electrode i, transmitted directly via the first connection or reconstructed from corresponding pertinent compressed signals transmitted via a second connection; then

- in a third step (310), receiving the pre-processed data $\varepsilon_i(t_N)$ of the signal acquired at the sampling time $t_N$, in the electrode i, the pre-processed data having been determined by the implementation of the pre-processing method defined according to any one of claims 1 to 8, and having been transmitted via a second connection; then

- in a fourth step (312), determining a second reconstruction term from the prediction function fi of the second term applied to the raw signals observed in the recent past up to the time $t_{N-p(i)}$, in the whole number m(i) of electrodes $\sigma_i(j)$ adjacent to the observed electrode i, or in the whole number m(i) of electrodes $\sigma_i(j)$ adjacent to the observed electrode i, and the observed electrode i, the observed raw signals being provided beforehand in the second provision step (308), then

- in a fifth step (314), reconstructing the raw data $u_i(t_N)$ of the signal acquired at the sampling time $t_N$, as being equal to the sum of the second reconstruction term and the pre-processed data $\varepsilon_i(t_N)$ of the signal acquired at the sampling time $t_N$, in the electrode i.

10. Decompression method according to claim 9, wherein
   the first and second connections are the same connection, or
   the first connection is a wired connection and the second connection is a wireless connection.

11. Unit for transmitting measurement data of electrocorticographic signals which evolve over time comprising a pre-processing data compression unit (28) for converting raw data $u_i(t_N)$ of electrocorticographic signal measurements collected by electrodes (6) at various locations on the surface of a cortex (24) into compressed pre-processing data $\varepsilon_i(t_N)$, and a transmission emitter (12) for transmitting compressed data over a connection,
   **characterised in that** the pre-processing compression unit (28) is configured to implement the effective pre-processing compression step defined according to any one of claims 1 to 8.

12. Data transmission unit according to claim 11, wherein the compression pre-processing unit (28) and the transmission emitter (12) are miniaturised to be implanted in the body of a patient, and the transmission emitter (12) is configured to transmit radioelectric waves.

13. System for acquisition and transmission of measurement data of electrocorticographic signals which evolve over time, comprising:

   - a set (4) of electrodes (6) which are arranged in direct contact with a cortex (24), each **characterised by** a different whole identification index i and a position r(i) at the surface of the cortex, the index i varying from 1 to L and L denoting the total number of electrodes (6), and each configured to acquire a raw electrocorticographic time signal $u_i(t)$; and
   - a sampling unit (8) which is configured to sample at successive times $t_N$, N being a whole number denoting the succession rank of a sample of a signal over time, the raw electrocorticographic time signals $u_i(t)$ and to encode their amplitude as associated raw data;
   - a pre-processing data compression unit (28) for converting the raw data $u_i(t_N)$ into pre-processed data $\varepsilon_i(t_N)$, at a lower entropy;
   - an entropic encoder (30); and
   - a transmission emitter (12) for transmitting the compressed pre-processed data;

   the system for acquisition and transmission being **characterised in that** the compression pre-processing unit (28) is configured to implement an effective step (204) for compression pre-processing of the raw data $u_i(t_N)$ which involves: for i varying from 1 to L and at each successive sampling time $t_N$, from a rank $N_0$, converting the raw signal $u_i(t_N)$ acquired by the electrode i into a pre-processed signal $\varepsilon_i(t_N)$ equal to the difference between a first term and a second term, the first term being equal to the raw signal $u_i(t_N)$ acquired at the electrode i at the current time $t_N$, and the second term being a prediction function fi which has no loss of information and which can be calculated in real time and which is temporally dependent on the raw signals $u_{\sigma_i(j)}(t_{N-k})$, $u_i(t_{N-k})$ observed in the recent past up to the time $t_{N-p(i)}$, p(i) designating an entire rank which is not zero of a most distant past relative to the rank N of the current time associated with the observed electrode i, k being a whole number varying from 1 to p(i), in at least one whole number m(i), different from L, of electrodes $\sigma_i(j)$ adjacent to the observed electrode i and at most in the whole number m(i) of electrodes $\sigma_i(j)$ adjacent to the observed electrode i and in the observed electrode i, $\sigma_i$ being a proximity function of the observed electrode i and $\sigma_i(j)$ denoting the indexes of the m(i) electrodes adjacent to the observed electrode i, j being a travel index of the proximity function $\sigma_i$ varying from 1 to m(i).

14. System for acquisition and transmission of measurement data of electrocorticographic signals according to claim 13, further comprising:

- a transmission receiver (14) which is remote from the transmission emitter (12), for receiving the compressed data,
- an entropic decoder (44), and
- a pre-processing decompressor (46) for converting the pre-processed data $\varepsilon i(t_N)$ into the raw data $u_i(t_N)$ which is configured to execute the steps defined according to any one of claims 9 and 10.

15. Computer product or program comprising a set of instructions configured to implement the compression method defined according to any one of claims 1 to 8 and/or the decompression method defined according to any one of claims 9 and 10 when they are loaded in and executed by a processor which forms the compressor and/or by a processor which forms the decompressor defined according to any one of claims 13 to 14.

FIG.1

FIG.2A

FIG.2B

FIG.2C

FIG.2D

202

204

206

FIG.3

FIG.4

FIG.5

302

304

N = N_0

306

308

310

312

314

318

N → N+1

316

non

oui

Ordre d'interruption
du procédé reçu ?

320

FIG.6

## RÉFÉRENCES CITÉES DANS LA DESCRIPTION

**Documents brevets cités dans la description**

- WO 2008015449 A

**Littérature non-brevet citée dans la description**

- **TAEHOON KIM et al.** Spatiotemporal compression for efficient storage and transmission of high-resolution electrocorticography data. *Proceedings of Engineering in Medicine and Biology Society (EMBC), 2012 Annual International Conférence of the IEEE,* 28 Août 2012, 1012-1015

- **SCHMALE S. et al.** Exploiting correlation in neural signais for data compression. *2014 22nd European Signal Processing Conférence, EURASIP,* 01 Septembre 2014, 2080-2084
- **CAPURRO IGNACIO et al.** Low-complexity, multichannel, lossless and near-lossless EEG compression. *2014 22nd European Signal Processing Conférence, EURASIP,* 01 Septembre 2014, 2040-2044